# EUROPÄISCHE PATENTSCHRIFT

(11) **EP 3 623 007 B1**
(45) Veröffentlichungstag und Bekanntmachung des Hinweises auf die Patenterteilung: **21.06.2023**
(21) Anmeldenummer: 19195190.4
(22) Anmeldetag: 03.09.2019
(51) Int. Cl.: A61N 1/375, F16J 15/3272, A61N 1/362, H01R 13/504, A61N 1/36, F16J 15/16

(54) **KONTAKTBUCHSE**
CONTACT SOCKET
PRISE DE CONTACT

(30) Priorität: 12.09.2018 EP 18194056
(43) Veröffentlichungstag der Anmeldung: 18.03.2020
(73) Patentinhaber: BIOTRONIK SE & Co. KG, 12359 Berlin (DE)
(72) Erfinder: Hartmann-Bax, Kathy, 14947 Nuthe-Urstromtal (DE); Lehmann, Stefan, 10245 Berlin (DE)
(74) Vertreter: Biotronik Corporate Services SE

(56) Entgegenhaltungen:
- EP-A2- 2 740 516
- CN-U- 205 350 358
- US-A1- 2004 122 481
- US-B2- 7 367 973

## Beschreibung

Die vorliegende Erfindung betrifft eine Kontaktbuchse, insbesondere zur Verwendung in einem Verbindungskopf (Header) eines implantierbaren Geräts.

Isolierende Dichtelemente in steckbaren Kontaktbuchsen in Verbindungsköpfen bzw. Headern von implantierbaren Geräten sind im Stand der Technik bekannt. Der Header dient hierbei insbesondere dazu, elektrische Impulse von Kern des implantierbaren Geräts zu einer in den Header gesteckten Elektrodenleitung zu leiten, ggf. auch in die umgekehrte Richtung. Die Bestandteile des Headers, insbesondere die Kontaktbuchse, werden typischerweise in einen Block aus Epoxidharz gegossen, um diese vor den Einflüssen der Zielumgebung zu schützen. Die eingangs genannten Dichtelemente sollen insbesondere eine Potentialtrennung zwischen den elektrisch leitenden Kontakten im Header gewährleisten. Weiterhin können solche Dichtungselemente als Anbindungsfläche für das oben genannte Epoxidharz sowie zur Herstellung eines bestimmten Abstandes zwischen den elektrisch leitenden Kontakten dienen.

Beispielsweise offenbart US 2008/0177167 A1 isolierende Dichtelemente in steckbaren Kontaktbuchse, welche aus einem Kern bestehen, der zur Wahrung der Distanz zwischen zwei elektrischen Kontakten, als Anschlag für die Montage, als Anbindungsfläche für Fügestoffe und der mechanischen Fixierung einer Dichtung ("wiper seal") dient. Die offenbarten Materialien für diesen Kern sind Polykristalline, einfache Kristalline oder aber kristallines Saphir.

Der generelle Aufbau von steckbaren Kontaktbuchsen in medizinischen Implantaten ist beispielsweise in den Dokumenten US 2015/0018909 A1, US 8,751,002 B2 oder US 2009/0017668 A1 offenbart.

Weiterhin beschreibt das Dokument US 2011/0059639 A1 die Verwendung von Silikon zur Dichtung und zur elektrischen Isolation in Bezug auf Kontaktbuchsen für medizinische Implantate. US 2009/0017668 A1 beschreibt den Gebrauch von *liquid silicone rubber* (LSR) für isolierende Dichtelemente in steckbaren Kontaktbuchsen.

Dokument EP 2740516 offenbart den Oberbegriff von Anspruch 1.

Ungeachtet dessen besteht nach wie vor ein Bedarf an geeigneten Designs für solche Dichtelemente, welche die oben genannten Aufgaben so gut wie möglich erfüllen.

Es ist daher basierend auf diesem Hintergrund eine Aufgabe der vorliegenden Erfindung, eine verbesserte Kontaktbuchse zur Verfügung zu stellen.

Diese Aufgabe wird durch eine Kontaktbuchse mit den Merkmalen des Anspruchs 1 gelöst. Vorteilhafte Ausgestaltungen sind in den entsprechenden abhängigen Ansprüchen und der folgenden Beschreibung dargestellt.

Ein Dichtungsring zur Verwendung in einem Header eines implantierbaren Geräts wird zur Verfügung gestellt. Der Dichtungsring weist hierbei die folgenden Komponenten auf:
- einen Außenring bzw. Kern, welcher einen thermoplastischen Hochleistungskunststoff umfasst oder im Wesentlichen daraus besteht, und
- einen Innenring, welcher ein Flüssig-Silikon oder ein Polyurethan umfasst oder im Wesentlichen daraus besteht,
wobei der Innenring und der Außenring zueinander formschlüssig angeordnet sind.

Es ist insbesondere vorgesehen, dass der thermoplastische Hochleistungskunststoff ausgewählt wird, aus der Gruppe umfassend ein Polyaryl und ein Flüssigkristallpolymer, wobei das Polyaryl insbesondere ausgewählt wird aus einem Polyarylethersulfon, einem Polyaryletherketon und einem Polyphenylensulfid.

Der Begriff "Flüssigkristallpolymer" wird im Sinne der vorliegenden Erfindung im dem Fachmann bekannten und geläufigen Sinne verwendet. Ein "Flüssigkristallpolymer" bezeichnet insbesondere ein aromatisches Polymer, dass in der Schmelze oder in Lösung hochgeordnete bzw. kristalline Bereiche aufweist. Nicht-beschränkende Beispiele umfassen aromatische Polyamide wie Aramid (Kevlar) und aromatische Polyester wie ein Polykondensat aus 4-Hydroxybenzoesäure und 6-Hydroxynaphthalen-2-carbonsäure (Vectran).

Als Polysulfon bzw. Polyarylethersulfon im Sinne der vorliegenden Erfindung wird insbesondere ein thermoplastischer Kunststoff mit einer Aryl-SO₂-Aryl Untereinheit bezeichnet. Nicht beschränkende Beispiele für Polysulfone bzw. Polyarylethersulfon umfassen Polysulfon (CAS Nr. 25135-51-7), Polyarylensulfon, Polybisphenylsulfon (25135-51-7), Victrex HTA (CAS Nr. 121763-41-5), Polyethersulfon (CAS Nr. 25608-63-3) und Polyphenylensulfon (CAS Nr. 25608-64-4).

Als Polyaryletherketon im Sinne der vorliegenden Erfindung wird insbesondere ein thermoplastischer Kunststoff mit einer Aryl-O-Aryl-C(O)-Aryl Untereinheit bezeichnet, also Arylreste, die immer abwechselnd über eine Ketogruppe (-C(O)- und eine Ethergruppe (-O-) miteinander verknüpft sind. Ein nicht-beschränkende Beispiel ist Polyetheretherketon (PEEK, CAS Nr. 29658-26-2).

Als Polyphenylensulfid im Sinne der vorliegenden Erfindung wird insbesondere ein thermoplastischer Kunststoff mit einer Aryl-S-Aryl Untereinheit bezeichnet, also Arylreste, die jeweils über eine Sulfidgruppe (-S-) miteinander verknüpft sind. Ein nicht-beschränkende Beispiel ist Poly(thio-p-phenylen) (CAS Nr. 26125-40-6, 25212-74-2).

Mit Vorteil ermöglichen die oben genannten Polyaryle und das Flüssigkristallpolymer eine effiziente Anbindung zu Harzen, beispielsweise Epoxid, die typischerweise in Header von implantierbaren Medizingeräten verwendet werden.

Als Flüssig-Silikon oder Flüssigsilikonkautschuk (*liquid silicone rubber*) im Sinne der vorliegenden Erfindung wird insbesondere ein thermoplastisches Elastomer bezeichnet, welches neben Poly(organo)siloxanen, vernetzbare Gruppen wie etwa Vinylgruppen und Si-H-Gruppen umfasst, die typischerweise in einer Edelmetall-katalysierten Reaktion addiert werden. Daneben kann das Flüssig-Silikon Additive wie verstärkende Stoffe und/oder Füllstoffe beinhalten.

Vorzugsweise handelt es sich beim dem Polyurethan um ein thermoplastisches oder elastisches (Elastomer) Polyurethan.

In einigen Ausführungsformen des Dichtungsrings ist vorgesehen, dass der Innenring mindestens eine Nut ausbildet, wobei die Nut einen Boden und zwei gegenüberliegende Seiten aufweist, die sich vom Boden erstrecken, und der Außenring in die Nut formschlüssig eingreift.

In einigen Ausführungsformen des Dichtungsrings ist vorgesehen, dass der Außenring mindestens einen Durchbruch aufweist, insbesondere ein Langloch, und der Innenring mindestens einen Steg aufweist, der sich von der einen Seite der Nut zur anderen Seite der Nut erstreckt, und der mindestens eine Steg den mindestens einen Durchbruch durchgreift.

In einigen Ausführungsformen des Dichtungsrings ist vorgesehen, dass der Außenring zwei oder mehrere Durchbrüche bzw. Langlöcher aufweist, insbesondere 3 Durchbrüche oder Langlöcher, und der Innenring entsprechend zwei oder mehrere Stege aufweist, insbesondere 3 Stege aufweist, wobei jeder der zwei oder mehreren Stege jeweils einen der zwei oder mehreren Durchbrüche durchgreift.

In einigen Ausführungsformen des Dichtungsrings ist vorgesehen, dass der Außenring spritzgegossen wird.

In einigen weiteren Ausführungsformen des Dichtungsrings ist vorgesehen, dass der Innenring spritzgegossen wird.

In einigen weiteren Ausführungsformen des Dichtungsrings ist vorgesehen, dass der Innenring um den Außenring spritzgegossen wird. Sofern der Außenring mindestens einen Durchbruch aufweist, bildet sich auf diese Weise vorteilhafterweise der mindestens eine Steg des Innenrings, welche durch den mindestens einen Durchbruch des Außenrings greift, und der Außenring und der Innenring zueinander formschlüssig angeordnet werden.

In einigen Ausführungsformen des Dichtungsrings ist vorgesehen, dass das Flüssig-Silikon ausgewählt wird aus einem Zweikomponentengemisch in beliebiger Shore-Härte, welches biokompatibel ist. Gemäß einem Ausführungsbeispiel können die Komponenten aus der Gruppe SILASTIC^{®} BioMedical Grade Liquid Silicone Rubbers (Dow Corning Corporatioin) oder Silpuran 6600 (Wacker Chemie AG) gewählt werden.

Eine Kontaktbuchse für ein implantierbares Medizingerät, insbesondere für einen Verbindungskopf eines implantierbaren Medizingeräts, wird zur Verfügung gestellt. Die Kontaktbuchse umfasst dabei mindestens einen Dichtungsring und eine Vielzahl von elektrischen Kontakten.

Als Verbindungskopf oder Header im Sinne der vorliegenden Erfindung wird insbesondere eine Baugruppe eines implantierbaren Medizingeräts bezeichnet, die eine elektrische Verbindung zwischen einer stromerzeugenden bzw. stromgebenden Komponente (Generatorkomponente, Batterie) oder einer stromerfassenden Komponente (Diagnosekomponente) eines implantierbaren Geräts und einer Elektrodenleitung gewährleistet.

Insbesondere umfasst die erfindungsgemäße Kontaktbuchse eine Vielzahl von elektrischen

Kontakten, wobei jeweils zwischen zwei elektrischen Kontakten ein Dichtungsring angeordnet ist. Dabei bilden die Vielzahl der elektrischen Kontakte und die dazwischen angeordneten Dichtungsringe einen Hohlraum, insbesondere einen zylindrischen Hohlraum, der zur Aufnahme eines Steckers einer Elektrodenleitung ausgebildet ist. Weiterhin sind die elektrischen Kontakte dazu ausgebildet, elektrische Ströme bzw. Impulse auf den Stecker der Elektrodenleitung zu übertragen oder davon zu empfangen. Typischerweise stehen die elektrischen Kontakte dafür in Kontakt mit einer stromerzeugenden bzw. stromgebenden Komponente (Generatorkomponente, Batterie) oder einer stromerfassenden Komponente (Diagnosekomponente).

Vorzugsweise handelt es sich bei den elektrischen Kontakten der erfindungsgemäßen Kontaktbuchse um Federkontakte. Die Kontaktbuchse kann am Ende oder am Anfang des Elektrodeneingangs auch eine Steckeraufnahme (Connector block) mit Gewindestift aufweisen. Solche Steckeraufnahmen können in gleicher Weise wie die Federkontakte durch einen Dichtungsring elektrisch isoliert und abgedichtet werden.

Weiterhin wird vorzugsweise die erfindungsgemäße Kontaktbuchse in einen Block aus einem duroplastischen Kunststoff gegossen, wobei dabei ein Verbindungskopf gebildet wird. Vorzugsweise handelt es sich bei dem duroplastischen Kunststoff um einen transparenten Kunststoff, insbesondere ein Epoxidharz.

Weiter wird ein implantierbares Medizingerät zur Verfügung gestellt, welches eine Kontaktbuchse umfasst.

Bei dem implantierbaren Medizingerät handelt es sich insbesondere um einen Herzschrittmacher, einen Kardioverter-Defibrillator oder einen Neurostimulator wie etwa einen Rückenmarksstimulator.

Bei einem implantierbaren Kardioverter-Defibrillator (ICD) handelt es sich insbesondere um eine Vorrichtung, die einen Stimulationsteil (Impulsgenerator) und einem Diagnostikteil (zur Erkennung bedrohlicher Rhythmusstörungen) aufweist, wobei der Stimulations-/Diagnostikteil, meist unter der Haut in der Nähe des linken Brustmuskels implantiert, über eine Elektrodenleitung mit der rechten Herzkammer verbunden wird. Die Elektrodenleitung wird hierbei typischerweise über die obere Hohlvene in die rechte Herzkammer geführt.

Bei dem Rückenmarksstimulator handelt es sich insbesondere um eine Vorrichtung zur Behandlung von chronischen neuropathischen Schmerzen, in der ein Impulsgenerator über eine Elektrodenleitung mit dem zu behandelnden Teil des Nervensystems verbunden ist, beispielsweise mit dem Hinterstrang des Rückenmarks.

Typischerweise weist das implantierbare Medizingerät ein Gehäuse aus einem beständigen, biokompatiblen Material auf, vorzugsweise Titan oder eine Titanlegierung, wobei im Gehäuse insbesondere Gerätekomponenten angeordnet sind, die zur bestimmungsgemäßen Verwendung des Gerätes erforderlich sind, wie beispielsweise Steuereinheiten, Energiequellen, Impulsgeneratoren oder Diagnoseeinheiten.

Ein Verfahren zur Herstellung eines Dichtungsrings wird zur Verfügung gestellt.

Dieses Verfahren fällt nicht unter den Schutzumfang der Erfindung und dient lediglich der Veranschaulichung. Das Verfahren umfasst die Schritte:
- Bereitstellen eines Außenrings, welcher ein thermoplastisches Hochleistungskunststoff umfasst oder im Wesentlichen daraus besteht,
- Bereitstellen eines Innenrings, welches ein Flüssig-Silikon oder ein Polyurethan umfasst oder im Wesentlichen daraus besteht, und
- Formschlüssiges Anordnen des Außenrings und des Innenrings zueinander.

Es ist insbesondere vorgesehen, dass der thermoplastische Hochleistungskunststoff ausgewählt wird, aus der Gruppe umfassend ein Polyaryl und ein Flüssigkristallpolymer, wobei das Polyaryl insbesondere ausgewählt wird aus einem Polyarylethersulfon, einem Polyaryletherketon und einem Polyphenylensulfid.

In einigen Ausführungsformen des Verfahrens ist vorgesehen, dass der Innenring mindestens eine Nut ausbildet, wobei die Nut einen Boden und zwei gegenüberliegende Seiten aufweist, die sich vom Boden erstrecken, und der Außenring in die Nut formschlüssig eingreift.

In einigen Ausführungsformen des Verfahrens ist vorgesehen, dass der Außenring mindestens einen Durchbruch aufweist, insbesondere ein Langloch, und der Innenring mindestens einen Steg aufweist, der sich von der eine Seite der Nut zur anderen Seite der Nut erstreckt, und der mindestens eine Steg den mindestens einen Durchbruch durchgreift.

In einigen Ausführungsformen des Verfahrens ist vorgesehen, dass der Außenring durch ein Spritzverfahren bereitgestellt wird, wobei insbesondere der Außenring nach dem Spritzen geschliffen oder gereinigt wird.

In einigen Ausführungsformen des Verfahrens ist vorgesehen, dass der Innenring durch ein Spritzverfahren bereitgestellt wird.

In einigen Ausführungsformen des Verfahrens ist vorgesehen, dass der Innenring um den Außenring gespritzt wird und dabei formschlüssig zu dem Außenring angeordnet wird.

In einigen Ausführungsformen des Verfahrens ist vorgesehen, dass der Außenring vor dem formschlüssigen Anordnen, insbesondere vor dem Umspritzen des Innenrings, vorbehandelt wird, insbesondere unter Verwendung eines Plasma oder eines Haftvermittlers (Primern).

Weitere Einzelheiten und Vorteile der Erfindung sollen durch die nachfolgende Figurenbeschreibung von Ausführungsbeispielen anhand der Figur erläutert werden.

Es zeigen:
- Fig. 1: schematische Darstellungen einer Ausführungsform des Dichtungsringes;
- Fig. 2: eine schematische Darstellung einer Ausführungsform des Herstellungsverfahrens; und
- Fig. 3: schematische Darstellungen einer Ausführungsform der erfindungsgemäßen Kontaktbuchse.

### Beispiele:

Bereitgestellt wird ein Dichtelement 100 aus einer PSU Komponente 10 und einer Umspritzung 20 aus LSR (*liquid silicone rubber,* Flüssig-Silikon). Hierbei ist die PSU Komponente insbesondere als ein Außenring 10 ausgebildet, und die LSR Umspritzung als ein Innenring 20.

Der PSU-Kern 10 erfüllt dabei folgende Funktionen und weist folgende Vorteile auf:
- Distanz zwischen 2 elektrischen Kontakten
- Anschlag für den Montageprozess
- Anbindungsfläche für Harz zur Bildung des Headers
- mechanische Fixierung zum Silikon (Durchbrüche)
- Anbindungsfläche für Silikon ggf. für Plasmaprozesse oder Primern

Die LSR-Umspritzung 20 erfüllt dabei folgende Funktionen und weist folgende Vorteile auf:
- Potentialtrennung zwischen 2 elektrischen Kontakten (gegeben durch stirnseitigen balligen Dichtflächen des Innenrings, die beim Spannen der Kontaktbuchse zu einer formschlüssigen Verbindung zwischen den elektrischen Kontakten führt)
- Potentialtrennung zwischen Elektrode und Kavität
- Abdichtung gegen Eindringen von Harz beim Harzverguss (realisiert durch die definierte Anschlagsfläche und daraus resultierenden technischen Nullspalt zwischen dem PSU Ring und dem Gehäuse des elektrischen Kontakts) Es handelt sich dabei um drei unabhängige Dichtfunktionen.

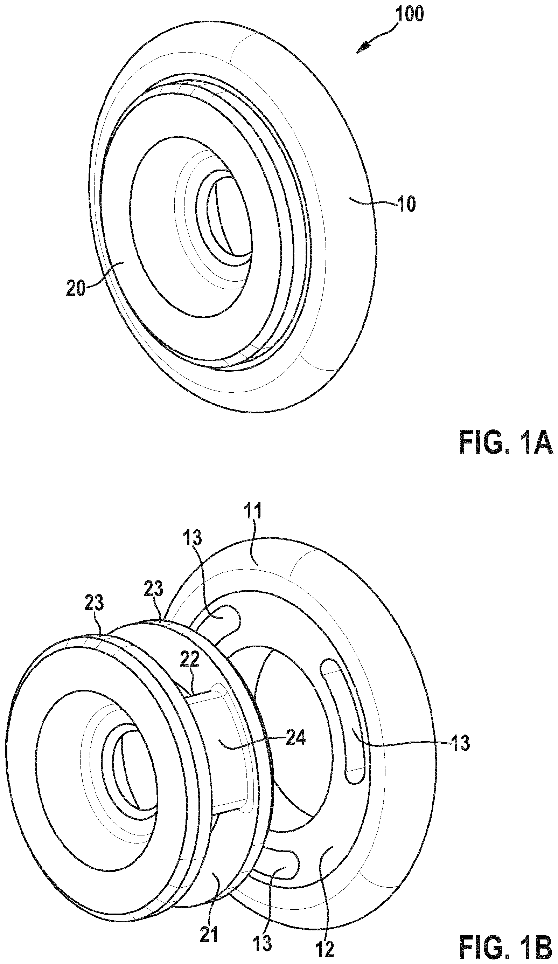

Figur 1A illustriert eine Ausführungsform des Dichtungsrings 100, welcher aus einem Außenring 10 aus PSU und einem Innenring 20 aus einem Flüssig-Silikon bzw. Flüssigsilikonkautschuk besteht. Figur 1B zeigt jeweils eine Detaildarstellung des Außenrings 10 und des Innenrings 20. Der Außenring 10 weist Langlöcher 13 (Durchbrüche) auf, die zur Verkrallung des Innenrings 20 aus LSR dienen. Der Innenring 20 wiederum bildet eine Nut 21 aus, welche einen Boden 22 und zwei gegenüberliegende Wände umfasst, die sich vom Boden erstrecken. Vorteilhafterweise weist der Außenring 2 oder 3 Stege auf, die sich von einer Wand 23 der Nut 21 zur anderen Wand 23 der Nut 21 erstrecken. Diese Stege 24 durchgreifen die jeweils dazugehörigen Langlöcher des Außenrings 10 und gewährleisten somit eine formschlüssige Verbindung zwischen Innenring 20 und Außenring 10.

Der Außenring 10 dient insbesondere als Anschlag zur Montage der elektrischen Kontakte 30. Aufgrund des verwendeten PSU ist eine besonders gute Anbindung zum Epoxidharz möglich, welche im Zuge der Herstellung des Headers verwendet wird, und somit zur elektrischen Isolierung oberhalb des PSU-Kerns beiträgt. Zusätzlich dient der Außenring als Führung der Verdrahtungsbänder zwischen den elektrischen Kontakten 30, beispielsweise eines 8-pol Moduls, zur Durchführung zum Inneren des Gehäuses. Dies unterstützt den Schweißprozess der Verdrahtungsbänder auf den elektrischen Kontakt 30. Per Design ist eine Potentialtrennung der Verdrahtungsbänder damit ebenso umgesetzt.

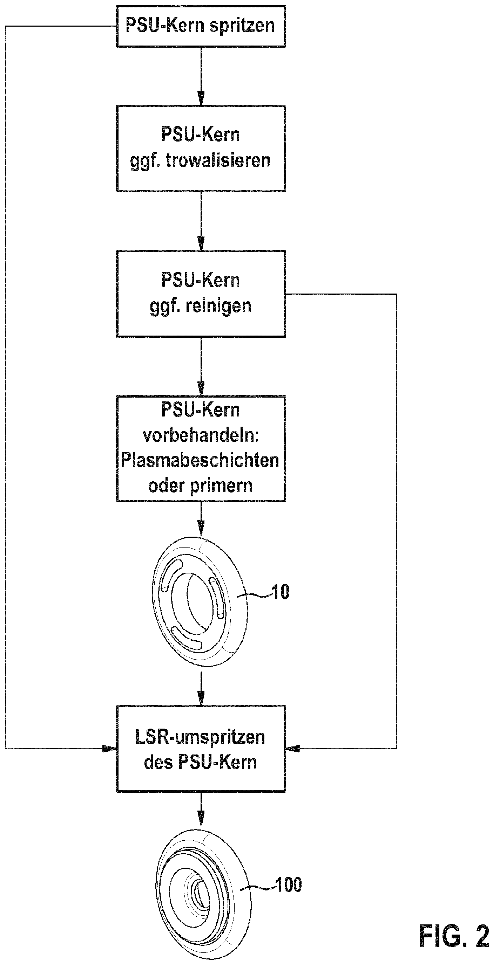

Fig. 2 stellt ein Fließschema der Herstellung des Dichtungsringes 100 dar. Diese Herstellung fällt nicht unter den Schutzumfang der Erfindung und dient lediglich der Veranschaulichung.

Zunächst wird der PSU-Kern bzw. Außenring 10 durch ein Spritzverfahren bereitgestellt. Ggf. wird der Außenring 10 durch Gleitschleifen (Trowalisieren) oder durch eine Reinigung vorbehandelt. Vorzugsweise wird der Außenring zur besseren Haftung des LSR 20 zusätzlich plasmabearbeitet oder geprimert (Behandlung mit einem Haftvermittler). Anschließend wird LSR um den Außenring 10 gespritzt, wobei das LSR dann den Innenring 20 bildet. Sofern der Außenring 10 Langlöcher 13 aufweist, werden diese vom LSR ausgefüllt, wobei auf diese Weise ein Innenring 20 mit Nut 21 und Stegen 24 entsteht, wobei die Stege 24 dann die jeweiligen Langlöcher 13 durchgreifen, und eine formschlüssige Verbindung zwischen Außenring 10 und Innenring 20 entsteht.

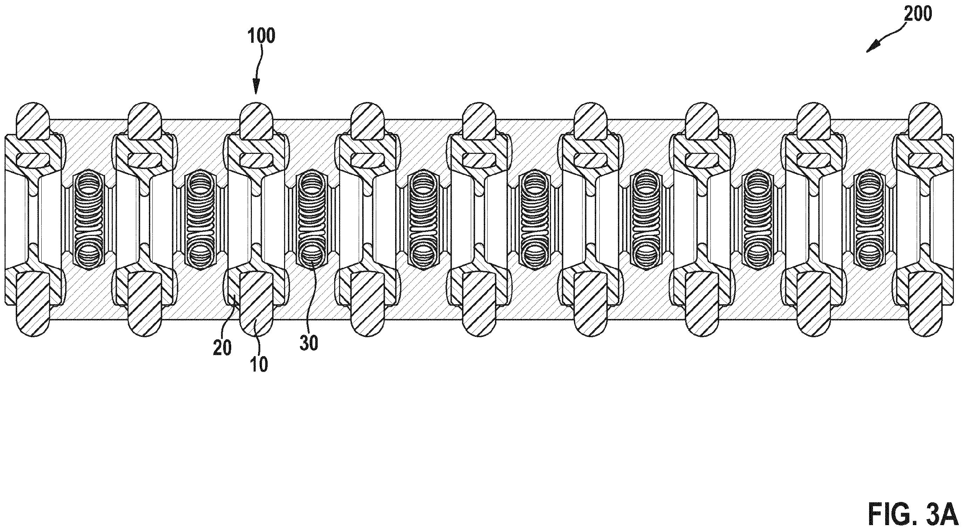

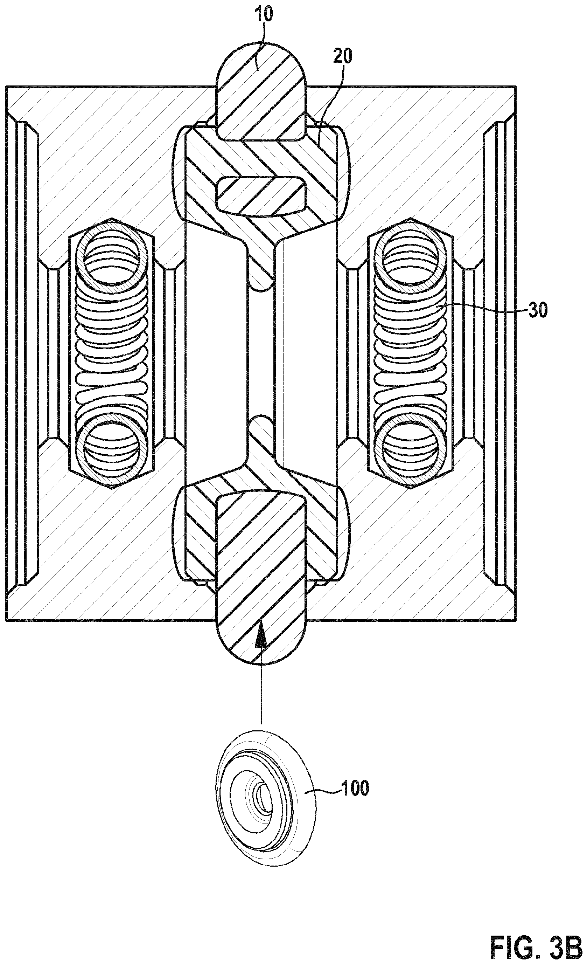

Fig. 3 zeigt schematische Ansichten einer erfindungsgemäßen Kontaktbuchse 200. Die Kontaktbuchse weist eine Vielzahl von elektrischen Kontakten 30 auf, insbesondere Federkontakte, zwischen denen jeweils ein Dichtungsring 10 angeordnet ist. Die Kontaktbuchse 200 weist im Inneren einen Hohlraum auf, insbesondere einen zylindrischen Hohlraum, der zur Aufnahme eines Steckers einer Elektrodenleitung ausgebildet ist. Hierbei dienen die elektrische Kontakte 30 zur Leitung eines Stromes oder elektrischen Impulses zwischen dem Stecker der Elektrodenleitung und den stromgebenden oder stromerfassenden Komponenten eines implantierbaren Geräts (beispielsweise ICD), die im Gehäuse des Geräts untergebracht sind. Die elektrische Kontakte 30 sind insbesondere über Verdrahtungsbändern mit dem Inneren des Gehäuses verbunden.

Zur Bereitstellung des Flüssig-Silikons wurden die Komponenten A und B das 40 Shore Material LSR Silastic 7-6840 (Dow Corning Corporation) im Verhältnis 1:1 miteinander gemischt und zur besseren Endvernetzung des Silikonmaterials im Umluftofen getempern (= post cure Prozess). Prinzipiell eigenen sich jedoch auch andere Shorehärten der LSR Silastic Produktfamilie z.B. 30,50 oder 60. Die Temperatur des Temperns ist dabei insbesondere vom Material des Kerns abhängig, insbesondere von dessen Schmelztemperatur bzw. Glasübergangstemperatur. Im Fall eines PSU-Kerns 10 wird bei einer Temperatur von etwa 150 °C für 12 Stunden getempert. Im Falle eines PEEK-Kerns 10 können höhere Temperaturen angewendet werden.

Alternativ können auch Flüssig-Silikone der Produktfamilie Silpuran 6600 mit unterschiedlichen Shorehärten zwischen 40 und 60 verwendet werden.

## Patentansprüche

1. Kontaktbuchse (200) für ein implantierbares Medizingerät umfassend eine Vielzahl
von elektrischen Kontakten und mindestens einen Dichtungsring (100);
jeder elektrische Kontakt der Vielzahl von elektrischen Kontakten aufweisend
- ein Gehäuse des jeweiligen elektrischen Kontakts;
der Dichtungsring aufweisend
- einen Außenring (10), welcher ein thermoplastischer Hochleistungskunststoff ausgewählt aus einem Polyaryl und einem Flüssigkristallpolymer umfasst oder im Wesentlichen daraus besteht, wobei das Polyaryl ausgewählt ist aus einem Polyarylethersulfon, einem Polyaryletherketon und einem Polyphenylensulfid, und
- einen Innenring (20), welches ein Flüssig-Silikon oder ein Polyurethan umfasst oder im Wesentlichen daraus besteht,
wobei der Innenring (20) und der Außenring (10) zueinander formschlüssig angeordnet sind, und der mindestens eine Dichtungsring (100) zwischen zwei elektrischen Kontakten der Vielzahl von elektrischen Kontakten angeordnet ist,
**dadurch gekennzeichnet,**
**dass** der Außenring (10) einen größeren Außendurchmesser als den Außendurchmesser des jeweiligen Gehäuses jedes der zwei benachbarten elektrischen Kontakte aufweist, und der Innenring (20) einen kleineren Außendurchmesser als den Außendurchmesser des jeweiligen Gehäuses jedes der zwei benachbarten elektrischen Kontakte aufweist.

2. Kontaktbuchse (200) nach Anspruch 1, **dadurch gekennzeichnet, dass** der Innenring (20) mindestens eine Nut (21) ausbildet, wobei die Nut (21) einen Boden (22) und zwei gegenüberliegende Seiten (23) aufweist, die sich vom Boden (22) erstrecken, und der Außenring (10) in die Nut (22) formschlüssig eingreift.

3. Kontaktbuchse (200) nach Anspruch 2, **dadurch gekennzeichnet, dass** der Außenring (10) mindestens einen Durchbruch (13) aufweist, insbesondere ein Langloch, und der Innenring (20) mindestens einen Steg (24) aufweist, der sich von der einen Seite (23) der Nut (21) zur anderen Seite (23) der Nut (21) erstreckt, und der mindestens eine Steg (24) den mindestens einen Durchbruch (13) durchgreift.

4. Kontaktbuchse (200) nach einem der vorherigen Ansprüche, **dadurch gekennzeichnet, dass** das Flüssig-Silikon ausgewählt wird aus einem Zweikomponentengemisch in beliebiger Shore-Härte, welches biokompatibel ist.

5. Kontaktbuchse (200) nach einem der vorherigen Ansprüche, **dadurch gekennzeichnet, dass** der Außenring (10) spritzgegossen wird.

6. Kontaktbuchse (200) nach einem der vorherigen Ansprüche, **dadurch gekennzeichnet, dass** der Innenring (20) um den Außenring (10) spritzgegossen wird.

7. Implantierbares Gerät umfassend eine Kontaktbuchse (200) nach einem Ansprüche 1 bis 6.

8. Implantierbares Gerät nach Anspruch 7, wobei das implantierbare Gerät als ein implantierbarer Kardioverter-Defibrillator oder ein implantierbarer Rückenmarksstimulator ausgebildet ist.

## Claims

1. A contact socket (200) for an implantable medical device, comprising a plurality of electrical contacts and at least one sealing ring (100);
each electrical contact of the plurality of electrical contacts comprising:
- a housing of the respective electrical contact;
the sealing ring comprising:
- an outer ring (10), which comprises or essentially consists of a high-performance thermoplastic material selected from a polyaryl and a liquid crystal polymer, the polyaryl being selected from a polyarylethersulfone, a polyaryletherketone and a polyphenylene sulfide; and
- an inner ring (20), which comprises or essentially consists of a liquid silicone or a polyurethane;
the inner ring (20) and the outer ring (10) being arranged with form fit relative to one another, and the at least one sealing ring (100) being arranged between two electrical contacts of the plurality of electrical contacts,
**characterized in that**
the outer ring (10) has a larger outside diameter than the outside diameter of the respective housing of each of the two adjoining electrical contacts, and the inner ring (20) has a smaller outside diameter than the outside diameter of the respective housing of each of the two adjoining electrical contacts.

2. The contact socket (200) according to claim 1, **characterized in that** the inner ring (20) has at least one groove (21), the groove (21) having a base (22) and two opposing sides (23) that extend from the base (22), and the outer ring (10) engaging in the groove (22) with form fit.

3. The contact socket (200) according to claim 2, **characterized in that** the outer ring (10) has at least one aperture (13), in particular an elongated hole, and the inner ring (20) has at least one web (24), which extends from one side (23) of the groove (21) to the other side (23) of the groove (21), and the at least one web (24) extends through the at least one aperture (13).

4. The contact socket (200) according to any one of the preceding claims, **characterized in that** the liquid silicone is selected from a two-component mixture having any Shore hardness, which is biocompatible.

5. The contact socket (200) according to any one of the preceding claims, **characterized in that** the outer ring (10) is injection-molded.

6. The contact socket (200) according to any one of the preceding claims, **characterized in that** the inner ring (20) is injection-molded around the outer ring (10).

7. An implantable device, comprising a contact socket (200) according to any one of claims 1 to 6.

8. The implantable device according to claim 7, wherein the implantable device is designed as an implantable cardioverter-defibrillator or an implantable spinal cord stimulator

## Revendications

1. Douille de contact (200) destiné à un appareil médical implantable, comprenant une multiplicité de contacts électriques et au moins une bague d'étanchéité (100) ; chaque contact électrique de la multiplicité de contacts électriques présentant :
- un boitier du contact électrique respectif ;
la bague d'étanchéité présentant
- une bague extérieure (10), laquelle comprend une matière plastique thermoplastique de performance élevée à base d'un poly aryle et un polymère à cristaux liquides, ou est essentiellement constituée, où le poly aryle est choisi parmi un poly aryle éther sulfone, une poly aryle éther cétone et un poly phénylène sulfure, et
- une bague intérieure (20), laquelle comprend un silicone liquide ou un poly uréthane, ou en est essentiellement constituée,
dans lequel la bague intérieure (20) et la bague extérieure (10) sont disposées en complémentarité des formes l'une par rapport à l'autre, et l'au moins une bague d'étanchéité (100) est disposée entre deux contacts électriques parmi la multiplicité de contacts électriques,
**caractérisée en ce que**
la bague extérieure (10) présente un diamètre extérieur plus grand que le diamètre extérieur du boitier respectif de chacun des deux contacts électriques voisins, et la bague intérieure (20) présente un diamètre extérieur plus petit que le diamètre extérieur du boitier respectif de chacun des deux contacts électriques voisins.

2. Douille de contact (200) selon la revendication 1, **caractérisée en ce que** la bague intérieure (20) forme au moins une rainure (21), où la rainure (21) présente un fond (22) et deux bords (23) latéraux opposés qui s'étendent à partir du fond (22), et la bague extérieure (10) s'insère par complémentarité des formes dans la rainure (22).

3. Douille de contact (200) selon la revendication 2, **caractérisée en ce que** la bague extérieure (10) présente au moins un passage (13), notamment un trou oblong, et la bague intérieure (20) présente au moins une tige (24) qui s'étend d'un côté (23) de la rainure (21) jusqu'à l'autre côté (23) de la rainure (21), et l'au moins une tige (24) passe à travers l'au moins un passage (13).

4. Douille de contact (200) selon l'une quelconque des revendications précédentes, **caractérisée en ce que** le silicone liquide est choisi à base d'un mélange à deux composants de dureté Shore quelconque qui est biocompatible.

5. Douille de contact (200) selon l'une quelconque des revendications précédentes, **caractérisée en ce que** la bague extérieure (10) est moulée par injection.

6. Douille de contact (200) selon l'une quelconque des revendications précédentes, **caractérisée en ce que** la bague intérieure (20) et la bague extérieure (10) sont moulées par injection.

7. Appareil implantable comprenant une douille de contact (200) selon l'une quelconque des revendications 1 à 6.

8. Appareil implantable selon la revendication 7, l'appareil implantable étant conçu sous forme d'un défibrillateur cardioverteur implantable ou d'un stimulateur de moelle épinière implantable.
